# EUROPEAN PATENT APPLICATION

(11) **EP 0 805 202 A1**
(43) Date of publication of application: **05.11.1997**
(21) Application number: 97106747.5
(22) Date of filing: 23.04.1997
(51) Int. Cl.: C12N 1/16, A21D 8/04, C12G 3/02, C12P 13/14, A23L 1/30

(54) **Yeast, and food and drink containing the same**

(30) Priority: 02.05.1996 JP 111398/96
(71) Applicant: Ajinomoto Co., Ltd., Tokyo (JP)
(72) Inventor: Nishimura, Yasushi, Ajinomoto Co., Inc., Kawasaki-ku, Kawasaki-shi, Kanagawa-ken (JP); Seguro, Katsuya, Ajinomoto Co., Inc., Kawasaki-ku, Kawasaki-shi, Kanagawa-ken (JP); Matsui, Kazuhiko, Ajinomoto Co., Inc., kawasaki-ku, Kawasaki-shi, Kanagawa-ken (JP); Izui, Hiroshi, Ajinomoto Co., Inc., Kawasaki-ku, Kawasaki-shi, Kanagawa-ken (JP)
(74) Representative: Strehl Schübel-Hopf Groening & Partner

(57) **Abstract**

The present invention provides yeast which accumulates glutamic acid in a cell, food and drink which are produced using the same, and a process for producing food and drink using the same.

A strain having a resistance to a glutamic acid antagonistic growth inhibitor is produced through mutation, and a strain in which a large amount of glutamic acid is accumulated in cells is separated therefrom. Food and drink are produced using the same. The glutamic acid antagonistic growth inhibitor is an agent in which growth of yeast is inhibited but this inhibition is antagonistically released by the co-existence of glutamic acid.

## Description

### Field of the Invention

The present invention relates to yeast which highly accumulates glutamic acid in a cell, and food and drink which are formed using this yeast, such as alcoholic drinks, bread products, fermentative seasonings and yeast extracts. Further, the present invention relates to a process for producing food and drink using this yeast.

### Prior Art

There are a wide variety of food and drink which are formed using yeast. Further, yeast contains vitamins, amino acids, minerals and the like in appropriate proportions, and besides it, yeast is a natural substance Accordingly, in recent years, people have increasingly eaten yeast itself with no safety concerns or have used an extract obtained from the yeast as a seasoning. However, these products have an odor ascribable to yeast and are lacking palatable taste. Therefore, these products are not said to have a good taste.

In order to eliminate these defects, there is a method in which breeding of yeast itself is improved to give yeast having a preferable taste or flavor, and food and drink are produced using the same. In a well-known method, alcoholic drinks are produced using yeast having a norvaline resistance [J. Brew. Soc. Japan, vol. 76, No. 12, 843-847 (1981)]. However, these drinks are to increase a flavor ingredient of esters or alcohols, not to change a palatable taste and the like.

Meanwhile, sodium L-glutamate (MSG), sodium 5'-inosinate (IMP) and sodium 5'-guanylate (GMP) are widely known to increase a palatable taste and the like, and these compounds are added to food and drink as seasonings according to the usage.

### Problems To Be Solved by the Invention

The present invention aims to improve a palatable taste and the like of food and drink which are formed using yeast accumulating higher amounts of L-glutamic acid.

### Means For Solving the Problem

The present inventors have assiduously conducted investigations to solve the above-mentioned problems, and have consequently found that a large amount of glutamic acid is accumulated in cells of edible yeast and that food and drink having much improved taste such as a palatable taste and the like can be produced using the yeast. These findings have led to the completion of the present invention. Specifically, it has been found that a large amount of glutamic acid is formed in cells by imparting a resistance to a chemical compound which was selected out of 95 chemical compounds as a glutamic acid antagonistic growth inhibitor, and that more tasteful food and drink such as alcoholic drinks, bread products, fermentative seasonings, yeast extracts and the like can be produced using the yeast.

That is, the present invention relates to:
(Invention 1) Yeast which exhibits a resistance to aspartic acid-β-hydroxamate or 4-fluorotryptophan and which accumulates glutamic acid in the cell.
(Invention 2) The yeast of Invention 1 which belongs to the genus Saccharomyces.
(Invention 3) The yeast of Invention 2 which is Saccharomyces cerevisiae.
(Invention 4) Food and drink which are formed using the yeast of Inventions 1 to 3.
(Invention 5) The food and drink of Invention 4 which are alcoholic drinks, bread products, fermentative seasonings or yeast extracts.
(Invention 6) A process for producing food and drink using the yeast of Inventions 1 to 3.

### Mode of Carrying Out the Invention

In order to obtain yeast that accumulates glutamic acid in cells in accordance with the present invention, induction through mutation can be utilized. Edible yeast is preferably a parent strain that undergoes mutation. Examples thereof include yeasts belonging to the genus Saccharomyces (Saccharomyces cerevisiae, Saccharomyces uvarum, Saccharomyces chevalieri, or Saccharomyces rosei), yeasts belonging to the genus Toluraspora (Toluraspora delbrueckii), yeasts belonging to the genus Kluyveromyces (Kluyveromyces thermotolerance), yeasts belonging to the genus Pichia (Pichia membranaefaciens), and yeasts belonging to the genus Candida (Candida utilis).

Of these, Saccharomyces cerevisiae is preferable. Beer yeast, wine yeast, sake yeast, whisky yeast, shochu (Japanese low-class distilled liquor) yeast and baker's yeast are especially preferable.

Mutation-inducing strains can be formed by appropriately applying to the above-mentioned yeasts of parent strains a known mutation-inducing method such as a method of irradiating ultraviolet light or radiation or a method of bringing yeast into contact with a chemical agent such as N-methyl-N'-nitro-N-nitrosoguanidine or ethyl methanesulfonate.

In order to obtain a strain in which a large amount of glutamic acid is accumulated in a cell from a mutation-inducing strain, yeast which exhibits a resistance to a glutamic acid antagonistic growth inhibitor is selected. The glutamic acid antagonistic growth inhibitor referred to in the present invention is an agent in which growth of yeast is inhibited but this inhibition is antagonistically released by the co-existence of glutamic acid. The present inventors have selected aspartic acid-β-hydroxamate and 4-fluorotryptophan out of 95 chemical compounds. Yeast which has underwent mutation is spread on a minimum medium containing this glutamic acid antagonistic growth inhibitor, colonies grown are separated, and mutants containing an increased amount of glutamic acid in the cells are selected from the colonies separated.

The yeast which accumulates a large amount of glutamic acid in cells can be used in food and drink which are ordinarily produced using yeast, such as alcoholic drinks, bread products, fermentative seasonings and yeast extracts. Further, it is also possible to eat the yeast cells per se as preparations or to use the same as feed. Especially, in the case of the yeast extract, glutamic acid accumulated in cells enhances a palatable taste of the yeast extract. Accordingly, it is preferable to use the product of the present invention in the yeast extract.

The use of yeast in the present invention means that yeast which is ordinarily used in the production of food and drink is replaced with the yeast of the present invention and that the yeast of the present invention is used in addition to yeast which is ordinarily used in the production of food and drink.

Food and drink can be produced by a conventional method. Alcoholic drinks can be produced by a method in which fruit of grapes, molasses, glucose or the like is directly fermented as a sugar source with yeast (single fermentation) or a method in which a starch of cereals such as rice, potatoes, buckwheat, corn and the like is saccharified with koji fungus, enzyme preparations or the like, and the alcohol fermentation is conducted in the presence of yeast (parallel fermentation). The above-obtained yeast can be used in these fermentations.

Bread can be made by a direct kneading method or an intermediate method. The former is a method in which all the raw materials are mixed from the beginning. The latter is a method in which yeast and water are first added to a part of a wheat flour to form an intermediate, and after the completion of the fermentation, the remaining raw materials are mixed therewith. A main starting material is a wheat flour and other starting materials are salt, fat, oil, water and yeast. Further, a sugar, a skim milk powder, a bread improving agent and the like are added as required. When using the above-obtained yeast as a starting material, a bread product having a strong, good taste can be obtained.

The yeast extract can be produced by a conventional method. That is, yeast is aerobically incubated in an ordinary medium containing a carbon source, a nitrogen source, inorganic substances, amino acids, and vitamins at a temperature of from 15 to 37°C, preferably 30°C, with a pH of from 3.0 to 10.0, preferably 7.0, and the cells are collected. Examples of the carbon source in the medium include glucose, sucrose and molasses. Examples of the nitrogen source include ammonia, ammonium salts such as ammonium sulfate, ammonium carbonate and ammonium acetate, urea, other nitrogen compounds, a yeast extract, and a corn steep liquor. Further, inorganic substances, amino acids and vitamins can be added thereto as required. The resulting cells are washed and suspended in water. Then, autolysis is conducted. In the autolysis, an autolysis accelerator or a cell wall lytic enzyme or the like may be added. The autolysis is carried out at a temperature of from 20 to 60°C, preferably 45°C, for from 10 to 40 hours, preferably 24 hours, with pH from 5.5 to 8.5, preferably 6.0. After the completion of the autolysis, the supernatant is concentrated through centrifugation, recovered as an extract, and dried through spray-drying. The yeast extract formed with yeast which accumulates glutamic acid in cells exhibits a strong palatable taste, improves a flavor, and increases a thick, full taste. Thus, it is quite a desirous yeast extract.

### Examples

The present invention is illustrated by referring to the following Examples.

### Example 1

### Screening of a glutamic acid antagonistic growth inhibitor:

A glutamic acid antagonistic growth inhibitor was screened by a paper disc method. That is, a paper disc having permeated therein 100 µl of a 100 µg/ml glutamic acid solution and a paper disc having permeated therein 100 µl of a 100 µ g/ml drug solution were placed at a fixed interval on a minimum flat medium (made by Difco, containing 0.67 % bacto yeast nitrogen base, 2 % glucose and 2 % agar) on which a parent strain had been widely spread, and the incubation was conducted at 30°C for 3 days. When the drug inhibited the growth of the parent strain, an inhibition circle was formed around the paper disc, and the degree of the growth inhibition was measured depending on the size of the circle. Further, when glutamic acid released the growth inhibition thereof, the parent strain was grown in the vicinity of the paper disc of glutamic acid to prevent formation of the inhibition circle. When glutamic acid and the drug are antagonistic in the portion where glutamic acid released the growth inhibition, the edge of the inhibition circle becomes linear. Ninety-five types of drugs were tested by this method. Consequently, 6-diazo-5-oxonorleucine, azaserine, aspartic acid-β-hydroxamate and 4-fluorotryptophan could be obtained as the glutamic acid antagonistic growth inhibitor.

### Example 2

### Screening of yeast having a resistance to a glutamic acid antagonistic growth inhibitor:

A parent strain, Saccharomyces cerevisiae AJ4005 which had been incubated in a YPD medium (containing 1 % yeast extract, 2 % peptone and 2 % glucose) at 30°C for 7 hours and baker's yeast, Saccharomyces cerevisiae AJ14710 were suspended in a 0.1 M phosphate buffer (pH 7.00) to a cell concentration of 10⁸ cells/ml.

Subsequently, methyl methanesulfonate was added to this suspension such that the final concentration reached 30 µm/ml. The mixture was allowed to stand at 30°C for 60 minutes, and the cells were collected through centrifugation. The cells were washed with a 6 % sodium thiosulfate aqueous solution and further with a 0.1 M phosphate buffer (pH 7.0). The resulting cells were incubated in a plate medium (0.67 % bacto yeast nitrogen base made by Difco, 2 % glucose and 2 % agar) containing 100 µg/ml of 6-diazo-5-oxonorleucine, 0.1 g/l of aspartic acid-β-hydroxamate or 0.1 g/l of 4-fluorotryptophan at 30°C for 7 days. Colonies formed were separated to obtain a strain having a resistance to each of a glutamic acid antagonistic growth inhibitor.

When selecting an excellent mutant, the cells were incubated in a YDP plate medium containing 1 % yeast extract, 2 % peptone, 2 % glucose and 2 % agar at 30°C for 24 hours, and inoculated in one platinum loopful of a GUM medium containing 4 % glucose, 1 % urea, 0.1 % KH₂PO₄, 0.05 % MgSO₄·7H₂0, 0.001 % NaCl, 0.001 % CaCl₂, 0.5 % yeast extract and 1.95 % MES buffer. The incubation was conducted at 30°C for 42 hours. Then, 1 ml of the thus-obtained culture solution was centrifuged to separate the cells from the supernatant. One milliliter of distilled water was added to the cells. The mixture was stirred well, and put in a hot-water bath of 90°C to extract free glutamic acid in the cells. The resulting extract was centrifuged to remove the extract residue. The amount of glutamic acid in the supernatant was then measured, and the strain containing the increased amount of glutamic acid was obtained. The amount of glutamic acid extracted was determined using a Bioteck Analyzer AS200 (manufactured by Asahi Chemical Industry Co., Ltd.). The 6-diazo-5-oxonorleucine-resistant strain induced from Saccharomyces cerevisiae AJ4005 was named AJ14709. The aspartic acid-β-hydroxamate resistant strain induced from Saccharomyces cerevisiae AJ4005 was named AJ14725. The 4-fluorotryptophan-resistant strain induced from Saccharomyces cerevisiae AJ4005 was named AJ14727. The 6-diazo-5-oxonorleucine-resistant strain induced from commercially available baker's yeast AJ14710 were named AJ14712. The aspartic acid-β-hydroxamate resistant strain induced from Saccharomyces cerevisiae AJ14710 was named AJ14726. The 4-fluorotryptophan-resistant strain induced from Saccharomyces cerevisiae AJ14710 was named AJ14728.

### Example 3

### Measurement of the resistance to each drug:

A sensitivity of the thus-obtained mutants and the parent strains to the drug is shown. That is, 6-diazo-5-oxonorleucine, aspartic acid-β-hydroxamate or 4-fluorotryptophan was added at a concentration shown in Table 1, Table 2 or Table 3 to 0.67 % bacto yeast nitrogen base made by Difco, 2 % glucose and 2 % agar to form a plate medium. The mutants and its parent strains were inoculated in this medium, and the growth at 30°C on Day 4 was observed. The results are shown in Table 1, Table 2 and Table 3.

**Table 1**

| Strain | 6-diazo-5-oxonorleucine (µg/ml) | | |
|---|---|---|---|
| | 10 | 50 | 100 |
| AJ4005 | - | - | - |
| AJ14709 | + | + | + |
| AJ14710 | - | - | - |
| AJ14712 | + | + | + |
| +: grown -: not grown | | | |

**Table 2**

| Strain | aspartic acid-β-hydroxamate (mg/ml) | | |
|---|---|---|---|
| | 0.05 | 0.1 | 1 |
| AJ4005 | - | - | - |
| AJ14725 | + | + | + |
| AJ14710 | - | - | - |
| AJ14726 | + | + | + |
| +: grown -: not grown | | | |

**Table 3**

| Strain | 4-fluorotryptophan (mg/ml) | | |
|---|---|---|---|
| | 0.05 | 0.1 | 1 |
| AJ4005 | - | - | - |
| AJ14727 | + | + | + |
| AJ14710 | - | - | - |
| AJ14728 | + | + | + |
| +: grown -: not grown | | | |

### Example 4

### Measurement of the amount of glutamic acid in a cell:

The amount of glutamic acid in the cell of each strain was measured. In the same manner as in Example 2, glutamic acid in the cell was extracted, and the amount thereof was measured. The results are shown in Table 4. AJ14725 and AJ14726 which were obtained as aspartic acid-β-hydroxamate resistant strains accumulate more glutamic acid in the cell body than AJ14709 and AJ14712 which were obtained as 6-diazo-5-oxonorleucine resistant strains.

**Table 4**

| Strain | DCW (g/liter) | Glu in a cell (g/liter) | (a)/(b) x 100 (%) |
|---|---|---|---|
| AJ4005 | 10.27 | 0.24 | 2.35 |
| AJ14709 | 11.48 | 0.48 | 4.18 |
| AJ14725 | 10.93 | 0.99 | 9.08 |
| AJ14727 | 10.38 | 0.92 | 8.82 |
| AJ14710 | 8.86 | 0.43 | 4.88 |
| AJ14712 | 7.42 | 0.74 | 9.94 |
| AJ14726 | 8.16 | 1.35 | 16.52 |
| AJ14728 | 8.79 | 1.16 | 13.17 |
| DCW: dry cell weight Glu: glutamic acid | | | |

### Example 5

### Production of yeast extract:

Autolysis was conducted using the strains to produce yeast extracts. First, each strain was incubated in a YPD plate medium for 24 hours. One platinum loopful of the strain was inoculated into 1L of GUM medium, and incubated at 30°C for 42 hours. The resulting culture solution was centrifuged, and the strain obtained was washed with 0.9 % of sodium chloride aqueous solution. The cells washed were collected, and distilled water was then added thereto to form a yeast slurry (cell concentration 20%). Subsequently, ethyl acetate was added thereto such that the final concentration reached 2%. The mixed solution was allowed to stand at 45°C for 24 hours for autolysis. The autolysis solution was centrifuged to remove the precipitate, and the residue was freeze-dried to form a yeast extract dry powder. The Glu content, the extract yield and the glutathione (GSH) content of the yeast extract dry powder were measured, and the results are shown in Table 3. It is noteworthy that the AJ14709 strain had the higher GSH content than the AJ4005 parent strain.

**Table 5**

| Strain | Glu in a cell (%) | Glu in an extract (%) | GSH in an extract (µl/liter) | DCW (%) | Extract weight (g) | Extract yield (%) |
|---|---|---|---|---|---|---|
| AJ4005 | 2.35 | 6.51 | ND | 10.27 | 4.44 | 43.2 |
| AJ14709 | 4.18 | 8.96 | 16.93 | 11.48 | 5.05 | 44.0 |
| AJ14725 | 9.08 | 16.39 | ND | 10.93 | 4.89 | 44.7 |
| AJ14727 | 8.82 | 11.31 | 1.97 | 10.38 | 5.17 | 49.8 |
| AJ14710 | 4.88 | 7.06 | 1.74 | 8.86 | 4.71 | 53.2 |
| AJ14712 | 9.94 | 16.28 | 1.74 | 7.42 | 4.32 | 58.2 |
| AJ14726 | 16.52 | 22.66 | 2.16 | 8.16 | 4.21 | 51.6 |
| AJ14728 | 13.17 | 18.41 | ND | 8.79 | 4.82 | 54.8 |
| GSH: glutathione | | | | | | |

### Example 6

Extraction by hot water was conducted using the strains to produce yeast extracts. First, each strain was incubated in a YPD plate medium for one day. One platinum loopful of the strain was inoculated into 1L of GUM medium, and incubated at 30°C for 42 hours. The resulting culture solution was centrifuged, and the strain obtained was washed with 0.9 % of sodium chloride aqueous solution. The cells washed were collected, and distilled water was then added thereto to form a yeast slurry (cell concentration 5%). The mixed solution was allowed to stand at 70°C for 10 minutes and the yeast extract was extracted. The extract solution was centrifuged to remove the precipitate, and the residue was freeze-dried to form a yeast extract dry powder. The Glu content, the extract yield and the glutathione (GSH) content of the yeast extract dry powder were measured, and the results are shown in Table 6.

**Table 6**

| Strain | Glu in a cell (%) | Glu in an extract (%) | DCW (%) | Extract weight (g) | Extract yield (%) |
|---|---|---|---|---|---|
| AJ4005 | 2.35 | 8.25 | 10.27 | 2.89 | 28.1 |
| AJ14709 | 4.18 | 15.72 | 11.48 | 2.54 | 22.0 |
| AJ14725 | 9.08 | 32.39 | 10.93 | 3.01 | 27.5 |
| AJ14727 | 8.82 | 23.64 | 10.38 | 2.65 | 25.5 |
| AJ14710 | 4.88 | 19.79 | 8.86 | 2.24 | 25.3 |
| AJ14712 | 9.94 | 33.24 | 7.42 | 2.31 | 31.1 |
| AJ14726 | 16.52 | 38.22 | 8.16 | 2.09 | 25.6 |
| AJ14728 | 13.17 | 29.58 | 8.79 | 2.82 | 32.1 |
| GSH: glutathione | | | | | |

### Example 7

The organoleptic evaluation was conducted on the resulting yeast extracts. The dry powder was dissolved in distilled water such that the content thereof reached 2%, and sodium chloride was added such that the content thereof reached 0.7%. The odor, the flavor, the palatable taste and the thick, full taste were evaluated by five panelists. The results are shown in Table 7. It was found that the mutants exhibited excellent properties compared to the parent strain.

**Table 7**

| Strain | Odor | Flavor | Palatable taste | Thick, full taste |
|---|---|---|---|---|
| AJ4005 | - | - | - | - |
| AJ14709 | + | + | ++ | ++ |
| AJ14725 | + | + | ++ | ++ |
| AJ14727 | + | ++ | ++ | + |
| AJ14710 | - | - | - | + |
| AJ14712 | + | - | ++ | ++ |
| AJ14726 | + | + | ++ | ++ |
| AJ14728 | + | ++ | ++ | + |
| ++: excellent, +: good, -: common | | | | |

### Example 8

### Making of bread:

Bread was made using AJ14710, AJ14726 and AJ14728. The yeast was incubated as follows. That is, one platinum loopful of each yeast was inoculated into 50 ml of a YPD medium, and incubated at 30°C for 24 hours. After the completion of the incubation, the culture solution was added to the total amount (450 ml) of the GUM medium, and incubated at 30°C for 24 hours. The culture solution was collected, and centrifuged to collect cells. The cells were washed twice with a 0.9 % sodium chloride aqueous solution. Bread was made using the yeast on the basis of the following composition of ingredients by the following process.

### Composition:

| | |
|---|---|
| wheat flour (strong flour) | 100.0 g |
| sodium chloride | 2.0 g |
| mold dilute solution | 0.4 g |
| yeast | 2.0 g |
| water | 65.0 g |

### Process:

| | |
|---|---|
| mixing | L8M3 |
| kneading temperature | 25°C |
| flouring time | 28°C, 75%, 30 min. |
| benching time | 20 min. |

A bread dough prepared was molded, proved at 35°C and humidity of 70% for 70 minutes, and then baked at 210°C for 17 minutes.

According to the organoleptic test, the bread which was made using AJ14726 or AJ14728 was excellent in terms of the flavor and the palatable taste compared to the bread which was made using the parent strain.

Saccharomyces cerevisiae AJ14710 was listed as deposited at the National Institute of Bioscience and Human Technology of the Agency of Industrial Science and Technology (No. 3, 1-ban, Higashi 1-chome, Tsukuba city, Ibaraki prefecture, Japan, postal code 305) on April 30, 1996 under Deposit No. FERM P-15605.

Saccharomyces cerevisiae AJ14726 was listed as deposited under the Budapest Treaty at the National Institute of Bioscience and Human Technology of the Agency of Industrial Science and Technology (No. 3, 1-ban, Higashi 1-chome, Tsukuba city, Ibaraki prefecture, Japan, postal code 305) on April 9, 1997 under Deposit No. FERM BP-5903.

Saccharomyces cerevisiae AJ14728 was listed as deposited under the Budapest Treaty at the National Institute of Bioscience and Human Technology of the Agency of Industrial Science and Technology (No. 3, 1-ban, Higashi 1-chome, Tsukuba city, Ibaraki prefecture, Japan, postal code 305) on April 9, 1997 under Deposit No. FERM BP-5904.

## Claims

1. Yeast which exhibits resistance to aspartic acid-β-hydroxamate or 4-fluorotryptophan and which accumulates glutamic acid in the cell.

2. The yeast according to claim 1 which belongs to the genus Saccharomyces.

3. The yeast according to claim 2 which is Saccharomyces cerevisiae.

4. Saccharomyces cerevisiae having the accession number FERM BP-5903.

5. Saccharomyces cerevisiae having the accession number FERM BP-5904.

6. Food and drink which are formed using the yeast according to any of the claims 1 to 5.

7. The food and drink according to claim 6 which are alcoholic drinks, bread products, fermentative seasonings or yeast extracts.

8. A process for producing food and drink using the yeast according to any of the claims 1 to 5.
